# EUROPEAN PATENT SPECIFICATION

(11) **EP 1 272 676 B1**
(45) Date of publication and mention of the grant of the patent: **29.03.2006**
(21) Application number: 01921607.6
(22) Date of filing: 12.04.2001
(51) Int. Cl.: C12Q 1/70

(54) **HIV DETECTION ASSAY AND REAGENTS THEREFOR**
HIV-NACHWEISVERFAHREN UND REAGENZIEN DAFÜR
ESSAI DE PHENOTYPAGE ET REACTIFS Y RELATIFS

(30) Priority: 14.04.2000 GB 0009374
(43) Date of publication of application: 08.01.2003
(73) Proprietor: GLAXO GROUP LIMITED, Greenford, Middlesex UB6 ONN (GB)
(72) Inventor: KLEIM, Joerg-Peter, Stevenage, Hertfordshire SG1 2NY (GB); ROBINSON, Laurence, Henry, Stevenage, Hertfordshire SG1 2NY (GB)
(74) Representative: Thornley, Rachel Mary
(86) International application number: PCT/GB2001/001728
(87) International publication number: WO 2001/079542

(56) References cited:
- EP-A- 1 109 019
- WO-A-00/66774
- WO-A-00/73511
- WO-A-01/57245
- WO-A-97/27480
- MARTINEZ-PICADO JAVIER ET AL: "Human immunodeficiency virus type 1 cloning vectors for antiretroviral resistance testing." JOURNAL OF CLINICAL MICROBIOLOGY, vol. 37, no. 9, 1999, pages 2943-2951, XP000886935 ISSN: 0095-1137
- HERTOGS K ET AL: "A RAPID METHOD FOR SIMULTANEOUS DETECTION OF PHENOTYPIC RESISTANCE TO INHIBITORS OF PROTEASE AND REVERSE TRANSCRIPTASE IN RECOMBINANT HUMAN IMMUNODEFICIENCY VIRUS TYPE 1 ISOLATES FROM FROM PATIENTS TREATED WITH ANTIRETROVIRAL DRUGS" ANTIMICROBIAL AGENTS AND CHEMOTHERAPY, AMERICAN SOCIETY FOR MICROBIOLOGY, WASHINGTON, DC, US, vol. 42, no. 2, February 1998 (1998-02), pages 269-278, XP000946883 ISSN: 0066-4804 cited in the application

## Description

The present invention relates to methods for generating recombinant viruses from samples such as uncharacterised virus samples or clinical specimens and to the use of the viruses so generated in assays, predominantly for the purpose of detecting altered viral susceptibility to anti-viral drugs and reagents. The invention further relates to deoxyribonucleic acid (DNA) sequences applied in the new methods and assays. The methods and assays are adapted to detect resistant virus more rapidly, sensitively and accurately than known assays by taking into account additional compensatory mutations arising in nucleotide sequences other than those encoding the anti-viral drug target, and by requiring fewer recombination events to generate recombinant virus.

Resistance of human immunodeficiency virus type 1 (HIV-1) to anti-retroviral drugs presents a major challenge in the chemotherapeutic prevention of progression to Acquired Immunodeficiency Syndrome (AIDS). The acquisition of viral resistance is accentuated by the rapid turnover rate of the virus *in vivo* combined with the high error rate of the viral reverse transcriptase (RT) enzyme (Coffin, 1995; Ho *et al*., 1995; Mansky and Temin, 1995; Wei *et al.*, 1995). Thus, the current goal of anti-HIV-1 therapy is maximally to suppress replication of the virus so as to delay the appearance of drug-resistant variants and maintain healthy levels of CD4⁺ immune cells for as long as possible (Vandamme *et al.,* 1999). To this end, the more recently introduced therapies of HIV-1 infection usually involve combinations of three or more anti-retroviral drugs.

The use simultaneously of reverse transcriptase (RT) inhibitors and inhibitors of the viral protease (PR) enzyme (protease inhibitors or Pls) has led to the emergence of HIV-1 variants with resistance mutations in both the drug targets RT and PR. These mutations alter the structure and/or chemical affinities of the target enzymes such that their ability to interact with the drugs is altered or reduced and the drugs show diminished activity against the mutated virus. Drug resistance mutations can potentially occur in the drug target molecules of any other viruses, for example, mutations associated with reduced susceptibility to nucleoside analogues have been demonstrated in the thymidine kinases or DNA polymerases of herpesviruses (Kimberlin and Whitley, 1996; Balfour, 1999).

It can be valuable to monitor the emergence of drug-resistant virus as part of the management of infection and disease. For example, assays of the resistance phenotype of HIV-1 isolates play a major role in the management of disease by identifying the development of reduced susceptibility, cross-resistance or re-sensitization to the many therapeutic drugs which are available. The co-culture of peripheral blood mononuclear cells (PBMC) from infected subjects with uninfected donor PBMC is one method which has been used to isolate HIV-1 from subjects for phenotyping assays (Japour *et al.,* 1993). Co-culture of PBMC is not ideal for large scale regular application because it involves the isolation of fresh PBMC and the long culture times have been shown to select for minority or less drug-resistant variants (Kusumi *et al.,* 1992; Mayers *et al.,* 1998).

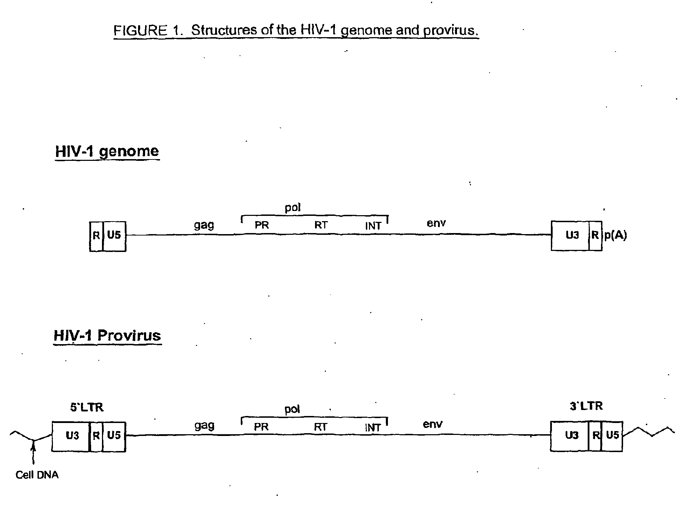

The recombinant virus assay (RVA) enables the reproducible determination of phenotypic susceptibility of HIV-1 from the subject's plasma (Kellam and Larder, 1994; Maschera *et al.,* 1995; Hertogs *et al.,* 1998) and has thus been instrumental in directing the choice of drugs used in HIV-1-therapy. In the RVA, HIV-1 RT and/or PR sequences are amplified from plasma by reverse transcription-polymerase chain reaction (RT-PCR) and co-electroporated into CD4⁺ cultured cells with a molecular clone of an RT and/or PR-deleted HIV-1 wild type HXB2 provirus, termed the 'vector' (a provirus is the infectious HIV-1 DNA sequence that is integrated into the host cell DNA, see fig. 1). The plasma-derived RT or PR sequences are inserted into the corresponding deletion site of the wild type vector by homologous recombination, and the resulting recombinant vector DNA inserts into the cellular DNA to yield full length infectious proviruses. Gene expression from the proviruses yields a population of viruses with a drug susceptibility phenotype representative of the subject's plasma viruses. The resulting virus stocks can be used for sensitivity tests against PR and/or RT inhibitors, depending on which part of the genetic information in the recombinant virus is derived from the clinical isolate. The RVA has several advantages over the co-culture of PBMC, in addition to being more rapid and reproducible. The production of virus in a common backbone enables a more accurate comparison of the drug sensitivities and growth characteristics of the viruses produced, and the shorter culture times minimize the outgrowth of minor variants.

The publication of Martinez-picado *et al.*, 1999 discloses plasmids and simple methods for rapid cloning of HIV-1 RT-PCR products from patient specimens and their use to generate infectious recombinant virus clones for virus phenotyping and genotyping. Eight plasmids with differing deletions of sequences encoding HIV-1 protease (PR), reverse transcriptase (RT), or Gag p7/p1 and Gag p1/p6 cleavage sites were constructed for cloning HIV-1 PCR products. Furthermore, a plasmid p83-10 which contains the complementary 3'-half HIV-1 genome including partial vpr and complete tat,rev,vpu,env, and nef genes as well as the 3' LTR is described. However, this backbone sequence (p83-10) which consist of the 3' half HIV-1 genome of a laboratory virus strain, is not used in a method for producing recombinant virus comprising recombination of the backbone sequence with the 5' LTR amplified from a patient sample.

Similarly, RVA-type assays can be directed to the detection of drug-resistant mutants of viruses other than HIV-1, by the recombination of sequences corresponding to the anti-viral drug target, derived by PCR or RT-PCR from a subject tissue sample, with a DNA vector including a wild-type viral genome carrying a deletion corresponding to the sequence encoding the drug target.

Mutants of HIV-1 with resistance to PR inhibitors frequently show reduced fitness as a consequence of inefficient cleavage of the precursor Gag and Gag-Pol polyproteins by the mutant PR enzyme (Maschera *et al*., 1995; Doyon *et al.,* 1996; Maschera *et al.,* 1996a, b; Mammano *et al.,* 1998; Pazhanisamy *et al.,* 1998; Zennou *et al.,* 1998). However, mutations which compensate for the cleavage defects can occur at the cleavage sites (CS) and function by improving viral fitness (Doyon *et al.,* 1996; Zhang *et al.,* 1997; Carrillo *et al.,* 1998; Mammano *et al.,* 1998; Alford *et al.,* 1999). Thus, mutations associated with reduced susceptibility to currently marketed anti-retroviral agents occur in at least three distinct regions of the HIV-1 genome: RT, PR and the CS.

Compensatory CS mutations have only been unambiguously demonstrated at the Gag p7/p1 and p1/p6 CS, however these sites are juxtaposed to the drug target genes PR and RT and have therefore been sequenced more frequently than the other CS. There are a total of nine CS present in the Gag and Gag-Pol polyproteins, all of which are potentially affected by PR mutations and hence are possible sites for compensatory mutations, thus it seems possible that uncharacterized CS mutations may occur at CS other than those covered in current PCR amplifications. There is some recent evidence for compensatory CS mutations occurring outside p7/p1 and p1/p6 (Mammano *et al*., 1998; Alford *et al*., 1999). Thus in order to truly represent the fitness and resistance of a virus as it occurs *in vivo*, viruses generated from clinical samples should ideally include all of the CS, both to facilitate the propagation of the virus and to minimize competition with fitter, potentially less resistant strains.

It can also be envisaged that, in HIV-1 or other viruses, compensatory mutations may occur in viral proteins associated with the RT enzyme in its functional role (accessory proteins), or in the binding, activation or initiation sites in the RNA genome from which the RT enzyme commences reverse transcription of the genome in the first step of viral replication. Similarly, in viral chemotherapy of certain infections, the viral polymerase enzyme is the drug target, in which circumstances resistance mutations may occur in the viral sequences encoding the polymerase and compensatory mutations might be envisaged in accessory proteins or in polymerase binding, activation, initiation etc. sequences of the viral genome. Other possible mechanisms by which viruses could overcome drug induced mutations which are deleterious to viral growth include the modulation of expression of those drug targets by compensatory mutations in regulatory nucleic acid sequences or by mutations affecting viral factors which control levels, timings or patterns of expression.

In an improvement to the RVA assay, described in International patent application No. PCT/GB/00/01639 (published as WO00/66774 plasma-derived viral sequences are recombined with a cloned wild type provirus carrying a deletion of at least a portion of the sequence encoding the antiviral drug target(s) and, additionally, a further deletion of sequences comprising potential sites of compensatory mutation, including the p7/p1 and p1/p6 CS. DNA vectors for use in such assays are also described in WO00/66774.

In a first aspect, the present invention provides a method for producing recombinant virus having the resistance profile of a virus sample by recombination of a nucleotide sequence derived from the virus sample with a nucleotide sequence ("backbone sequence") from a laboratory virus strain, to produce recombinant virus having the resistance profile of the virus sample, wherein the backbone sequence comprises a portion of the infectious nucleic acid sequence (INAS) of a wild type laboratory virus strain extending from a first site within or adjacent to either the 3' (three prime) or the 5' (five prime) terminus of the INAS, and the nucleotide sequence derived from the virus sample comprises substantially the remainder of the INAS. An INAS is defined as a nucleic acid sequence that is sufficient to produce viable virus after introduction into susceptible cells, in the case of HIV, the INAS is often referred to as the "provirus".

According to a second aspect, the present invention provides an assay for the detection and characterisation in a virus sample of virus resistant to an anti-viral drug, the assay comprising the steps of
(i) producing recombinant virus having the resistance profile of the virus sample by recombination of a nucleotide sequence derived from the sample of virus suspected of including drug-resistant virus with a nucleotide sequence ("backbone sequence") from a laboratory virus strain to produce recombinant virus having the resistance profile of the virus sample, wherein the backbone sequence comprises a portion of a laboratory virus INAS extending from a first site within or adjacent to either the 3' or the 5' terminus of the INAS, and the nucleotide sequence derived from the virus sample comprises substantially the remainder of the INAS;
(ii) incubating cells infected with the recombinant virus with an antiviral drug; and
(iii) detecting the viability of virus or of infected cells after incubation to determine the sensitivity of the recombinant virus to the drug.

The backbone sequence may comprise 20-90% of the laboratory strain INAS, for example 25-75%, preferably at least one third of the laboratory strain INAS. In particular embodiments, the backbone sequence is from a wild type laboratory virus strain and comprises approximately half of a wild type INAS, preferably the 3' half.

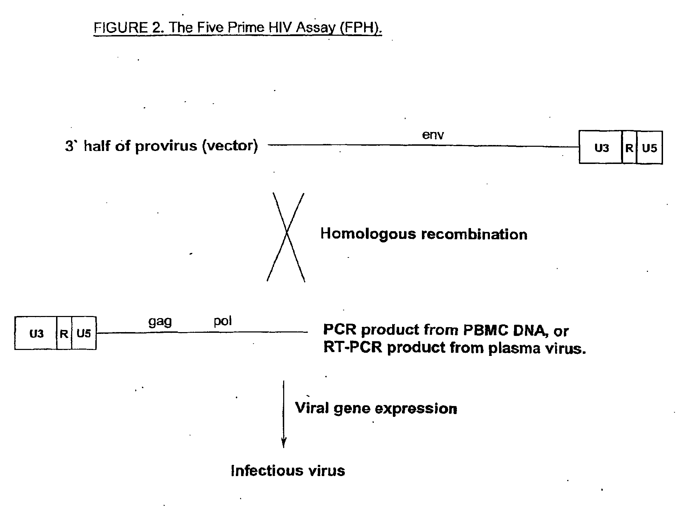

The phenotyping assay of the present invention may be a tissue culture-based assay in which recombinant infectious virus, e.g. HIV-1 is derived from a clinical sample. In particularly preferred embodiments, approximately half of the genetic information of the recombinant virus variant formed in the assay is derived from the clinical isolate, the remainder usually being derived from a standard laboratory viral strain, usually a wild type strain (the backbone sequence). Where the virus being tested for drug resistance is HIV-1, the standard laboratory strain preferably comprises the 3' end of the provirus (INAS), which includes the *env* gene. This is recombined in the cell culture with a sequence derived from a clinical isolate, which includes at least the sequence encoding one drug target. Preferably the sequence derived from the clinical isolate includes the PR drug target and the entire *gag* sequence. This enables all of the cleavage sites (CSs) in the gag region to be included in the resistance assay. In a particularly preferred embodiment, the sequence derived from the clinical isolate(s) includes the entire sequences encoding the PR and RT drug targets and all of the CS in Gag and Pol, i.e. from the N-terminal Gag p17/p24 junction to the C-terminal Pol RNAseH/INT site. The assay in this form is hereafter referred to as the FivePrime HIV assay (FPH) and is summarised in figure 2.

Recombination of the backbone sequence DNA, e.g. in preferred embodiments the 3' portion of the INAS, with the remainder of the INAS derived from the subject's plasma virus, results in the formation of infectious virus which can be used in drug sensitivity assays.

In a third aspect, the present invention provides a backbone DNA sequence comprising wild type laboratory strain sequences extending from a first site at or adjacent the 3' or the 5' terminus of an INAS to a second site within the INAS. Generally, the backbone sequence includes the 3' end of the viral genome and covers at least 25% of the genome, preferably at least one third of the genome. In particular embodiments, the backbone sequence comprises approximately half of the wild type laboratory strain virus genome; where the virus is HIV-1, this is preferably the 3' half of a provirus.

In a further aspect, the present invention provides a kit for the performance of a method according to the first aspect of the invention or an assay according to the second aspect of the invention, the kit comprising a backbone sequence according to the third aspect of the invention. Also provided is a backbone sequence according to the third aspect of the invention, for use in a method for producing recombinant virus, for example a method according to the first aspect of the present invention, and the use of a backbone sequence according to the third aspect of the present invention in an assay for the detection of virus resistant to an anti-viral drug.

Throughout this specification and the claims which follow, the term "adjacent" to the 3' or the 5' terminus shall be taken to mean at a site which is close enough to that terminus to allow the resulting sequence to produce viable recombinant virus after recombination in the method or assay of the present invention. It will be apparent to the skilled man how the proximity to the terminus will affect the viability of the resulting recombinant virus and he will be able to select an appropriate site from his own knowledge or by trial and error, without undue burden.

Preferably the two DNA sequences recombined in the method and assay of the invention each represent approximately half of the viral genome. The length of the HIV provirus can vary between strains, so the terms "half", "5' half" or "3' half' are approximate. In the case of HIV, where the term "half" is used it is meant that the *env* gene lies in the 3' half, which is generally derived from the laboratory virus strain and the gag, PR and RT sequences, including the protease cleavage sites encoded by these sequences, lie in the 5' half which is generally derived from the viral sample under study e.g. a patient sample. Conveniently, the division between the 3' and 5' halves of the HIV provirus can be made in the sequence encoding the viral integrase protein (INT), which results in approximately equal halves, but as INT may prove itself to be a suitable drug target or a site of resistance mutation, in certain embodiments of the invention it may be desirable to include the entire INT sequence in the patient-derived 5' "half".

The assay of the present invention has several advantages over existing technology in monitoring anti-HIV-1 drug resistance. One such advantage is that all the Gag protease cleavage sites (Gag PR CSs), i.e. from the N-terminal Gag p17/p24 junction to the C-terminal Pol RNAseH/INT site, may be included in the analysis. This may be achieved by the use of a DNA sequence which has all of these elements missing, e.g. a backbone sequence which is missing most of the 5' half of the provirus. This may be achieved using a backbone sequence which includes sequences covering only the 3' half of the HIV-1 provirus (INAS). This is recombined with plasma-derived sequences comprising the remainder of the HIV-1 provirus, i.e. the 5' half. Current RVA assays based on deleted proviral clones can only measure the potential effects of changes in PR, RT, and in the Gag p7/p1 and p1/p6 CSs, as these are the only sequences missing from the vectors used (Hertogs *et al*., 1998; Robinson *et al.,* 1999, 2000).

Furthermore, the present invention requires at least one fewer recombination event to generate a full-length provirus than do conventional assays. Conventional RVA assays require a minimum of four recombination events to produce a complete integrated provirus, because each end of the RT-PCR product derived from the patient's virus sample must be joined with the corresponding regions within the vector DNA, then each of the provirus termini must be joined with the cell genome in order to create an integrated recombinant provirus. However, in the FPH method of the present invention, there is only one region of overlap within the backbone sequence and therefore in total only three recombination events are required.

The reduction of the total number of DNA recombination events required upon introduction of the different viral DNA segments into susceptible cells will have a significant beneficial effect on i) the time-span between the transfection procedure and the detection of virus replication in the cell cultures and ii) the general likelihood of recovering virus from the experiment considerations which are becoming increasingly important with the reduced fitness of drug resistant strains. Since the timely reporting of sensitivity data can be crucial for treatment decisions for HIV-1 infected subjects, these parameters are important for drug-resistance phenotyping.

Where the methods or assays of the present invention are applied to HIV, sample (RT-)PCR products derived from infected subjects may be cotransfected with a backbone 3' PCR product or clone from any previously characterised strain of HIV-1 to produce viable virus, for example strains other than the standard HXB2 laboratory strain may be used. In this way, the FPH can easily be adapted to generate variants in different 'backbone' strains, which might be useful for examining the effects of the backbone on fitness, drug resistance or pathogenesis. The strain chosen to provide the backbone sequence should preferably be well characterised in order that variations in growth and sensitivity to antivirals in the recombinant virus produced using the backbone sequence can be assigned to the backbone strain or to the sequences derived from the patient sample. A wild-type strain is generally suitable as it contains no pre-existing resistance mutations. The backbone strain also needs to be chosen with regard to the cell culture conditions to be used in the generation of recombinant virus and the subsequent drug resistance assay or other uses of the recombinant virus produced. The skilled man will be able to select a backbone strain which displays sufficiently strong replication characteristics in the cell culture of interest. Thus, throughout this specification and the claims which follow, reference to a "laboratory strain", "laboratory virus strain", "wild type strain" or to a "wild-type laboratory virus strain" should be understood to mean any previously characterised viral strain.

As previously discussed, recombinant virus produced by the method of the present invention may be used in drug sensitivity assays. A suitable drug sensitivity assay is described in Pauwels *et. al.* (1988) and is an MTT-reduction colorimetric cell viability assay. Cells infected with the virus are plated into the wells of tissue culture plates and incubated with the anti-viral drug to be tested. After incubation, MTT (3-(4,5-dimethylthiazol-2-yl)-2,5-diphenyltetrazolium bromide) is added to the cell culture. Only live cells produce the blue formazan product after incubation with MTT. This product is detectable by reading the optical absorbance at 540-590nm, so the absorbance reading gives a measure of the cells which have been protected from the virus by the anti-viral drug. Viral drug resistance results in a decrease in surviving cells and a decrease in absorbance at 540-590nm.

In addition to the application of FPH in drug sensitivity assays, the characterization of viruses produced by FPH may extend our ability to determine novel *in vivo* molecular mechanisms of drug resistance. In addition to new mutations in the drug targets themselves and in the p7/p1 and p1/p6 CS, possible mechanisms of drug resistance might include compensatory mutations in CS other than p7/p1 and p1/p6 or in accessory factors or nucleic acid elements that interact with the drug targets. Other resistance-associated mutations may modulate the timing or intensity of gene expression of drug targets or of factors that regulate drug target activity, or may modify the activity of accessory factors by mechanisms which are not necessarily compensating for altered interaction with the drug targets. Use of the current FHP system of the present invention will allow the detection and characterisation of resistance-associated mutations which fall outside of the RT, PR and CS sequences utilised in the standard and improved RVA.

As with the RVA, recombinants generated by FPH are in a known background strain with consistent properties and env is kept constant, thus cell tropism is the same for any recombinant. Another advantage of the FPH is that the vector can be a PCR product or plasmid, there is therefore no requirement for complex deleted proviral plasmids. We have adapted the FPH to make site-directed mutants (see Examples below), which is a situation in which its increased efficiency should be an advantage for constructing mutants with reduced replicative capacity. Other applications include the generation, of recombinants in different background strains, simply by providing a PCR product of the 3' half of the required strain.

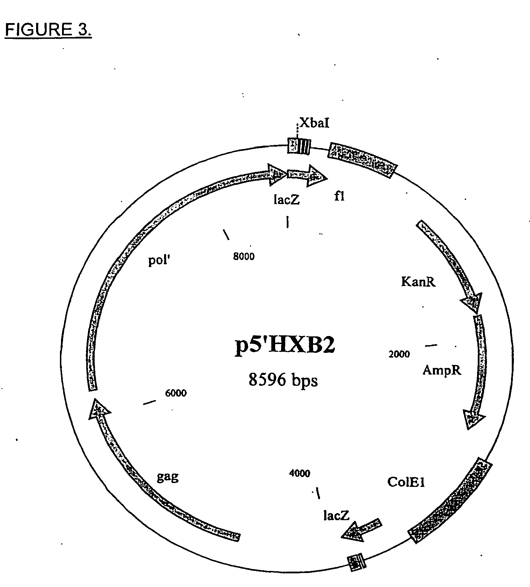

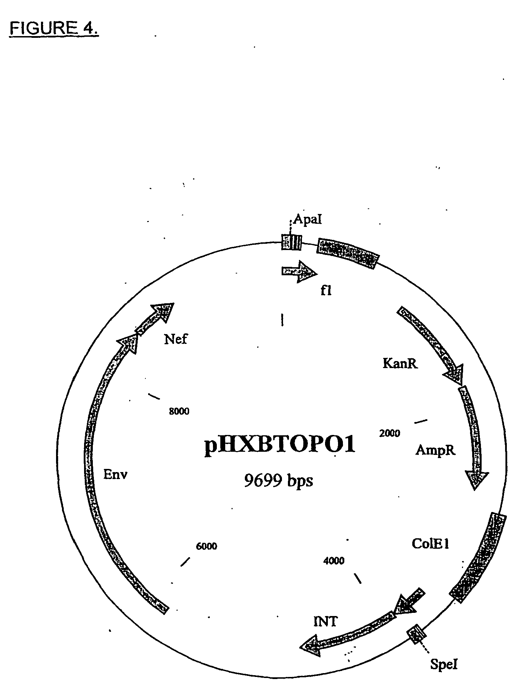

Recombinant virus production by the method of the present invention has applications other than the provision of virus for phenotyping and drug resistance studies. The greater efficiency of virus generation by the FPH method is an advantage for the generation of mutants by site-specific mutagenesis, especially for those mutants that have greatly reduced fitness which impede their propagation. For the purpose of generating mutants, a molecular clone of the 5' half of the provirus can be used as a template for site-directed mutagenesis (Fig. 3). Virus can be generated from the mutant clone by cotransfection with a plasmid clone of the 3' half (Fig. 4). Likewise a clone of the 3' half can be mutated and cotransfected with a cloned wild type strain 5' half to produce virus. Using a "reverse" FPH method, or 'Three-Prime HIV assay' (TPH), where the 3' fragment, including the env coding sequences, is from the isolate and the 5' fragment is from the vector, the FPH could also be used to examine immunological escape mutants for immunological studies. For example, in the case of HIV the 3' half of the viral genome contains the env gene, which shows the greatest immunological variability between isolates. Using the backbone strain to generate the 5' sequences and recombining this with a 3' half derived by PCR from the patient's viral sample will produce recombinant virus with predictable growth characteristics and displaying the immunological profile of the patient's viral population.

The experimental approach used in the FPH method for making recombinant virus can also be used for viral diseases other than HIV, provided that there is an in vitro replication system and the size of the INAS of the target virus permits PCR amplification. Subsequent assay utilising the recombinant virus could, as for HIV, include not only drug sensitivity assays with compounds directed against a specific viral enzyme, but also immunological tests, i.e. to determine variability in antigenic sites encoded in the PCR product derived from a clinical isolate.

The present invention is further described and illustrated in the following nonlimiting Examples and by reference to the accompanying drawings in which:
FIG. 1 shows the structures of the HIV-1 viral genome and provirus;
FIG.2 is an outline in diagrammatic form of the FivePrime assay as applied to HIV (FPH Assay);
FIG. 3 is a diagram of a plasmid containing the 5' half of the HXB2 provirus (p5'HXB2):
FIG.4 is a diagram of a plasmid containing the 3' half of the HXB2 provirus (pHIVTOPO1).
FIG. 5 is an outline in diagrammatic form of the PCR-mediated recombination of HIV-1 plasma-derived sequences.
FIG. 6 is a diagram of a plasmid construct containing the U3-R megaprimer (FPHmegaprim1).
FIG. 7 shows data from an experiment that compared the relative rates of production of virus in the FPH and RVA.
FIGs. 8 A-D show ethidium bromide-stained agarose gels of the PCR reaction products from Examples 1 to 4.
FIG. 9 shows an ethidium bromide-stained agarose gel of the PCR reaction products from Example 6.

The following examples illustrate how the FPH assay involves the amplification of the 5' half of the HIV-1 genome from clinical samples in a series of enzymatic steps designed to obtain a final ≈4.5 kilobase pair (kbp) PCR product. From Fig.1 it can be seen that this amplicon will contain i) the coding region for PR, ii) the coding region for the RT, and iii) the regions corresponding to all of the Gag and Gag-Pol protease CSs. The 3' half of the genome encompassing the *env* gene is derived from a standard laboratory strain of HIV-1 to facilitate virus replication in immortalized CD4+ cell lines. The 3' portion of the viral genome can be obtained either by PCR, or from a plasmid clone. Fig. 2 shows how the two portions of the HIV INAS are recombined by co-transfection into a suitable cell culture to give infectious virus. Titrated infectious virus stocks obtained upon co-transfection of both subgenomic fragments can be used in drug sensitivity assays. In the Examples, we demonstrate the use of PCR to obtain the 5' sequences required for the FPH assay, the production of recombinant virus from these sequences obtained from plasma virus and, finally, we demonstrate that virus is produced more rapidly by FPH than by the RVA.

### Examples

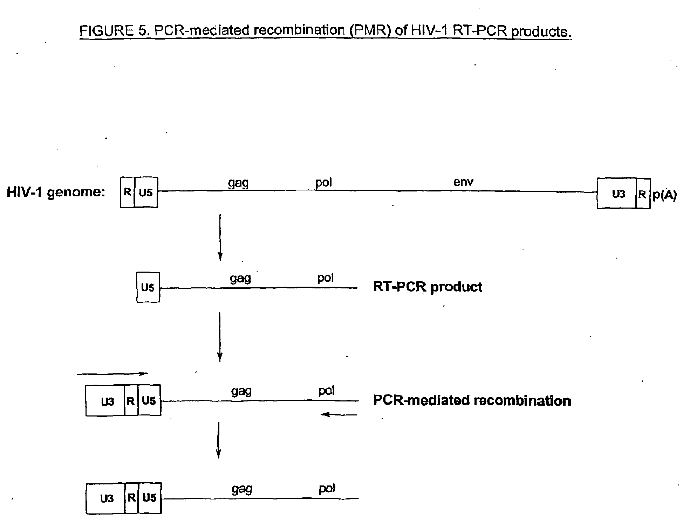

To construct infectious viruses reflecting ≈50% of the genetic information present in clinical isolates, we employed long range PCR to obtain the 5'half of the genome. This technique has been facilitated by the use of combinations of different thermostable polymerases (Lundberg *et al*., 1991; Bames, 1992; 1994). The following sections describe nucleic acid amplifications starting from peripheral blood mononuclear cells (PBMC), amplifying proviral DNA from within the cellular DNA, and also from plasma, using a coupled long range reverse transcriptase polymerase chain reaction (RT-PCR) amplification approach to amplify sequences from plasma virus. The promoter-containing U3 region is absent from the HIV-1 RNA genome (see Fig. 1). We therefore included an additional step to add this sequence (see Fig. 5), to ensure the resulting virus would be fully infectious (Fang *et al.,* 1999).

### Example 1: Long-range PCR of the 5'LTR-gag-pol region from PBMC DNA.

Purification of DNA from the PBMC of 13 subjects was carried out with the Qiagen QIAmp DNA Blood kit and amplified by 2 rounds of PCR using the Platinum*Taq* High Fidelity PCR kit from Gibco BRL. First round primers were: LTR-1 and INT-1; Second round primers were LTR-2 and INT-2 (Table 1). Thermal cycles used for the PCR were as follows: 1^{st} round: 94° for 30 seconds (s), (1 cycle); 93° for 10 s, 65° for 30 s, 68° for 4 minutes (m) 30 s, (10 cycles); 93° for 10 s, 60° for 30 s, 68° for 4 m 30s + 5 additional s for each consecutive cycle, (25 cycles). Second round: 94° for 30 s, (1 cycle); 93° for 10 s, 60° for 30 s, 68° for 4 m 30 s, (10 cycles); 93° for 10 s, 55° for 10 s, 68° for 4 m 30 s + 5 additional s for each consecutive cycle, (25 cycles).

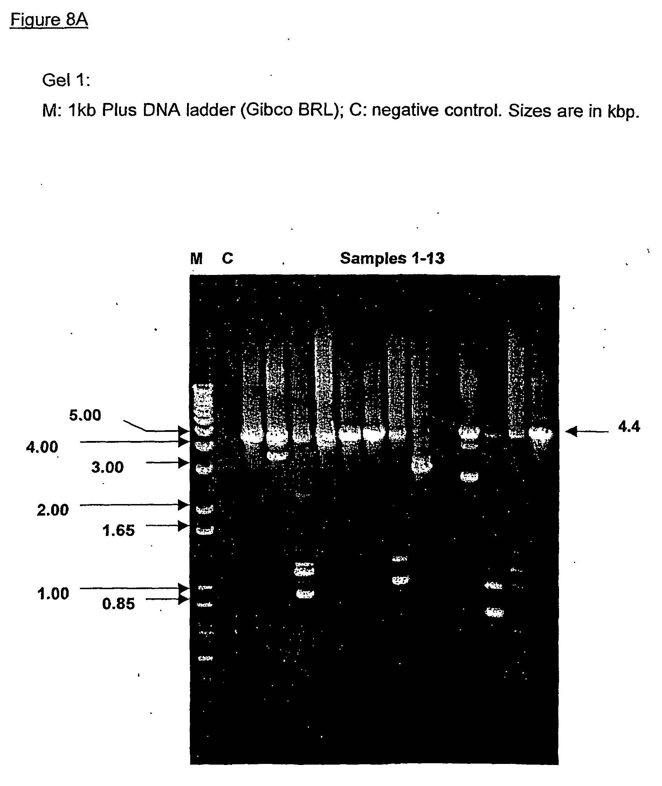

In Fig. 8A, an ethidium bromide-stained 0.7% (w/v) agarose gel is presented, showing electrophoretic separation of the DNA fragments (Gel 1). The desired 4.4 kbp HIV-1 LTR-*gag*-*pol* amplification product is indicated by the arrow on the right, the size of selected size markers are indicated on the left (LTR is an abbreviation of long terminal repeat). Note that despite the presence of incorrectly sized bands in some of the samples, the products are capable of producing virus in the FPH provided the 4.4 kbp fragment is present, thus at least 11/13 (85%) would be functional.

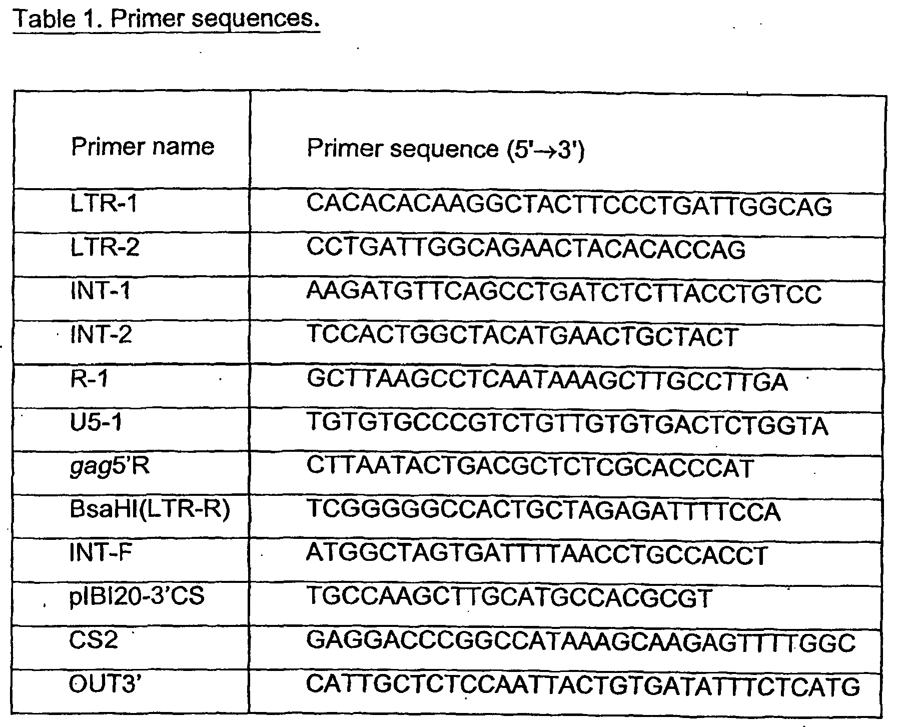

### Example 2: Long range RT-PCR of the gag-pol region from plasma

Plasma RNA was purified from 6 HIV-1-infected subjects using the Ultrasensitive Roche Amplicor HIV-1 Monitor Assay, and reverse transcribed using Superscript II RT (Gibco BRL). A reverse transcription reaction consisted of (in a total volume of 50 µl) 1× First Strand Buffer, 10 mM DTT, 500 nM primer INT-1, 500 µM each dNTP, 20 µg/ml BSA, 5% (v/v) DMSO, 40 units (u) of RNasin ribonuclease inhibitor (Promega), 200 u Superscript II RT, 20 µl RNA extract. The RT reaction was incubated at 45° for 45 m and then 50 µl of the following PCR mixture was added: 1x High Fidelity PCR buffer, 2 mM MgSO₄, 400 nM primer R-1, 3.5 u PlatinumTaq High Fidelity enzyme mixture. The sample was then subjected to the thermal cycling described for the first round amplification from PBMC DNA in Example 1. The Platinum *Taq* High Fidelity kit was used for the second round with 5 µl of the first round reaction and PCR primers U5-1 and INT-2. Thermal cycling was identical to the second round PCR from PBMC DNA.

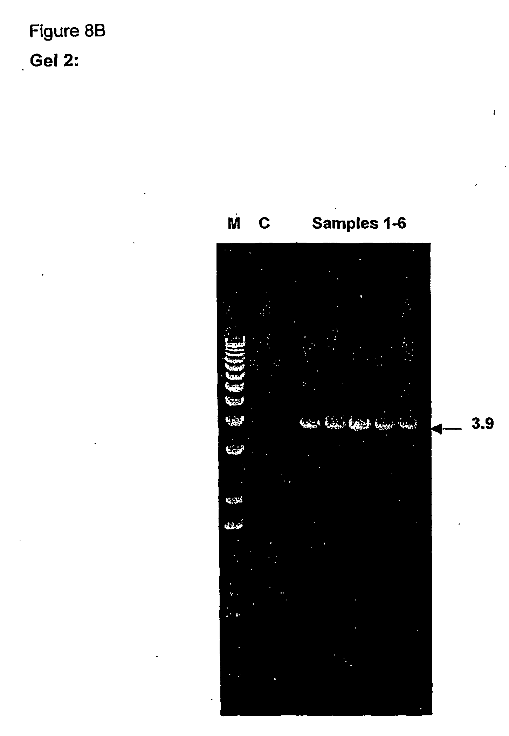

In the photograph of Fig. 8B, Gel 2, the 3.9 kbp HIV-1 fragment is indicated. Five out of six (83%) gave correctly sized products.

### Example 3: PCR-mediated recombination (PMR)

Due to the absence of the promoter-containing U3 region from the 5' sequences of the HIV-1 RNA genome (see figures 1 and 5), there is a risk that the RT-PCR products from plasma could have reduced infectivity. We therefore sought to add the U3 region by PCR-mediated recombination (PMR; Fang *et al.,* 1999). The U3 and R regions were amplified by PCR from the LTRs of a plasmid containing an RT-deleted HXB2 provirus, and the amplification product was used as a megaprimer for PMR (figure 5). The megaprimer was generated using *Pfu* polymerase (Stratagene) with the standard conditions recommended by the manufacturer and primers LTR-1 and gag5'R. After thermal cycling, 10 u of the methylated-site-specific restriction endonuclease Dpnl was added to each reaction and incubated at 37° for 4 hours to degrade the template DNA. Samples from ten 100 µl reactions were purified with the Qiagen Quiaquick PCR purification kit and pooled.

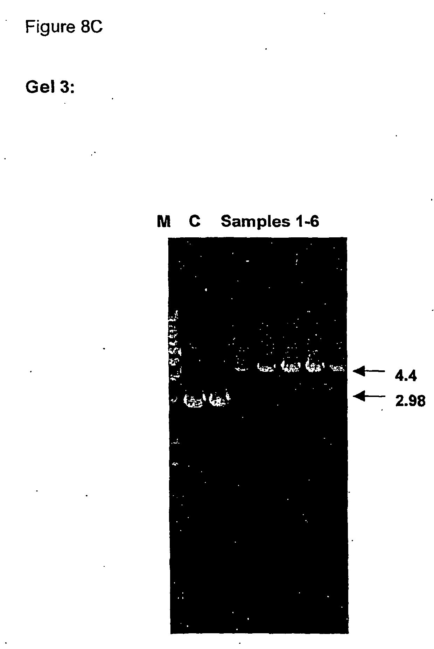

The PMR reactions were carried out using the PlatinumTaq High Fidelity PCR kit and (in 50 µl reactions) 10 µl megaprimer (≈ 1µg), 2 µl RT-PCR product (≈ 200 ng) and 200 nM primer INT2. Cycling was identical to the second round PCR of PBMC DNA. The reaction products are presented in Gel 3 (Fig. 8C).

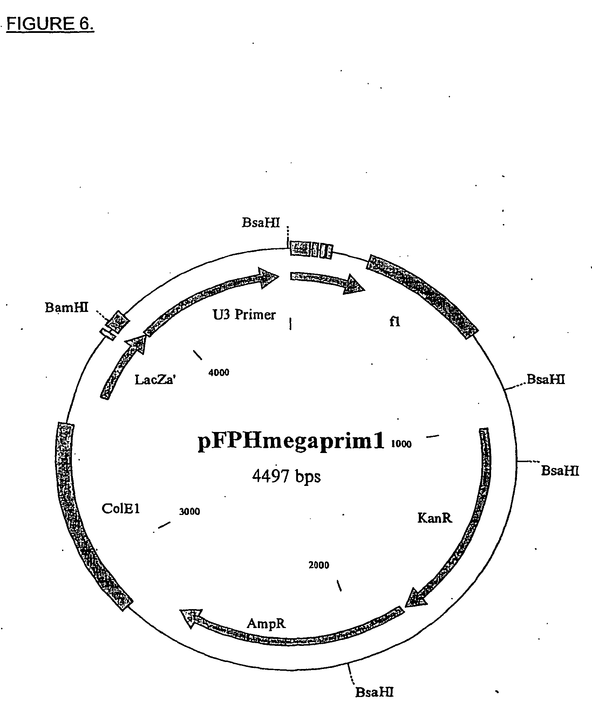

The reactions worked for all 5 samples that had generated RT-PCR products in example 2, showing the expected 4.4 kbp fragment expected from the addition of the U3 region. The products in the control and sample 1 are PCR products of the template plasmid used for generation for the megaprimer, suggesting that *Dpn*l digestion was insufficient to completely remove the template. Due to this contamination, we cloned the U3-R megaprimer into a backbone sequence plasmid and used this new plasmid as a template to generate the primer by PCR (Fig. 6). Alternatively, the primer can be cut from the plasmid with the restriction enzymes BamHI and *Bsa*HI and gel-purified before use.

### Example 4: Production of virus from plasma RNA by the FPH method

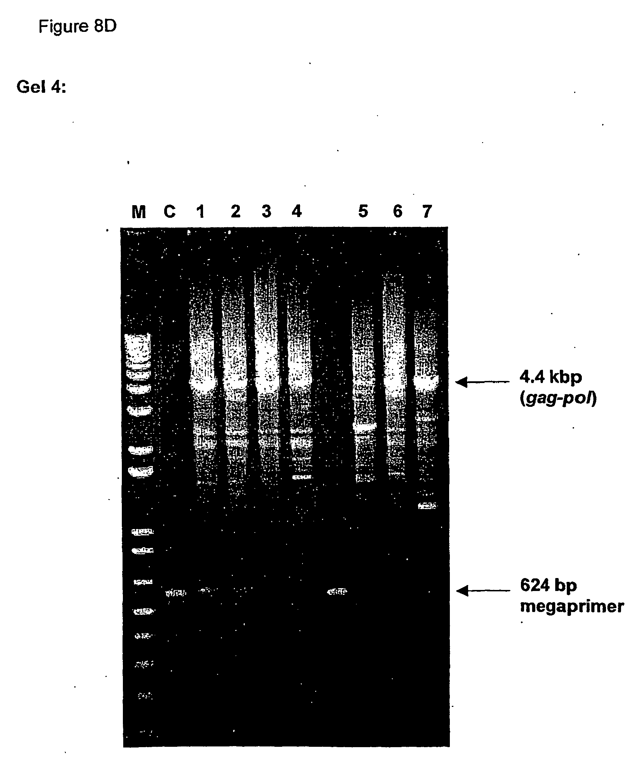

The megaprimer was generated by PCR of the template plasmid pFPHmegaprim1 (Fig. 6). The template was degraded by *Dpn*I digestion and the primer was then ready to use directly in a PMR reaction without any requirement for purification. A PMR reaction was carried out with seven plasma RT-PCR products, the products are shown in Gel 4 (Fig. 8D). In order to generate virus from the products, a plasmid was constructed which contained the 3' half of an HXB2 provirus (pHXBTOPO1; Fig. 4). This construct provided a convenient - method for generating virus upon cotransfection with FPH RT-PCR products. The cotransfections were carried out as described (Robinson *et al.,* 1999, 2000). Viral cytopathic effect was observed in all seven samples and they were harvested over a 9-14 day post-transfection time span.

### Example 5: Comparative efficiencies of FPH and RVA

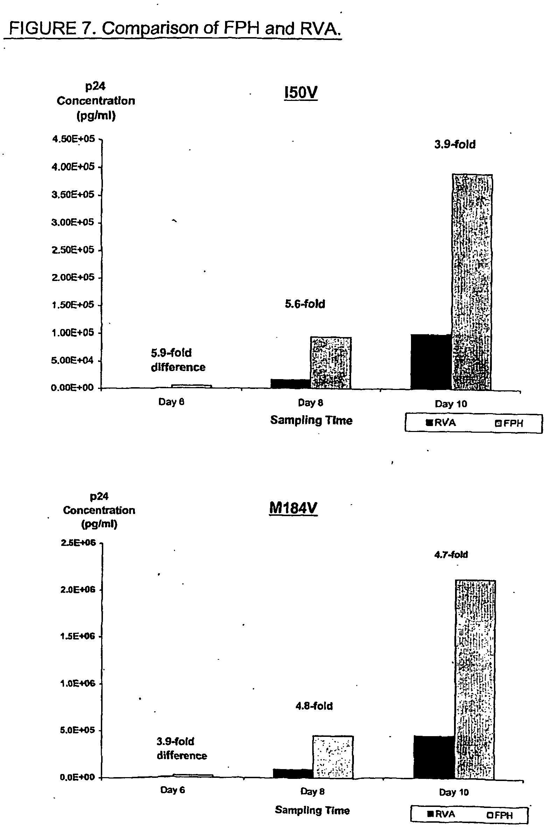

We examined the possibility that virus would be produced more rapidly by FPH compared to the RVA. Plasmid p5'HXB2 (Fig. 2) was mutated to introduce an 150V mutation in the PR gene, or an M184V mutation in RT. Both of these mutations lead to a reduction in viral fitness (Back *et al.,* 1996; Pazhanisamy *et al.,* 1998; Robinson *et al.,* 1999, 2000). A PCR product was generated from the mutated plasmids which covered the 5' half of the provirus (primers LTR-2 and INT-2 - table 1) or covered the CS, PR and RT deletion in the RVA vector pHXBΔCSPRTC (Robinson *et al*. 1999, 2000; primers CS2 and OUT3'). Both sets of PCR products had regions of overlap with their respective vectors. The.products were cotransfected into MT4 cells with 5 µg of the linearised vectors pHXBTOPO1 or pHXBΔCSPRTC, respectively. Each mutant was examined in triplicate by both FPH and RVA. Samples of the cell culture supernatants were taken at days 6, 8 and 10 post-transfection and the HIV p24 antigen concentrations in the sampled supernatants were determined by enzyme immunoassay, in order to give a quantitative indication of the appearance of virus (Murex HIV Mab kit, Abbott Diagnostics). The averaged results are presented in Figure 7. With both mutants, p24 levels were significantly higher in the FPH supernatants at all time points. With the I50V mutant, the differences ranged from 5.9-fold higher at day 6, to 3.9-fold higher at day 10. In the M184V samples, the p24 concentration differences ranged from 3.9-fold fold higher at day 6, to 4.8-fold higher at day 8. This data supports the theory that FPH is a more efficient method for generating virus than the RVA. The outcome of this experiment was dependent upon the amount of overlap between the PCR product and the vector. If the overlap was greater in the FPH than the RVA, due to the primers that were used to generate the PCR products, the efficiency of recombination rather than total number of recombination events could explain the observed difference in appearance in virus. However, the total overlap in the FPH samples was 182 nucleotides (nt), whereas in the RVA the overlap in the 5' region was 161 nt and the 3' overlap was 176 nt, thus the total regions of homology were greater in the RVA (total overlap of 337 nt). Therefore the most likely explanation for the relative rapidity of the FPH was the reduced number of recombination events required to reconstruct a whole provirus. In assays where the template virus has one or more compensatory mutation(s) upstream of the p7/p1 and p1/p6 CS, the upstream CS mutation(s) would be included in the FPH recombinants but absent in recombinants produced by RVA. In such cases one would predict that the FPH would be even more markedly rapid than the RVA.

### Example 6: Generation of virus from plasma by FPH.

In this experiment, plasma from three HIV-1 infected subjects experiencing virological failure on Pl-containing therapy regimens was used as the basis for the FPH to generate recombinant virus. Plasma virus from Subject 1 had an 154M mutation in the protease, which is a mutation associated with resistance to amprenavir (APV) (see table 2, below). Virus from Subject 2 had L10I, M46I, 150V and a Lp1'F mutation at the p1/p6 protease cleavage site, all of which are mutations typical of high level APV resistance. Isolate 3 had a series of mutations consistent with a combination of protease inhibitors used in therapy (indinavir (IDV) followed by APV), the L10I, L63P, A71V, V82A-and Ap2V at the p7/p1 protease cleavage site are typical of reduced IDV susceptibility and the L101, V321 and M46L are associated with both IDV and APV resistance pathways.

The 5' region of the genome was amplified from these plasmas by RT-PCR. Virions in up to 1 ml of plasma were pelleted from an HIV-1 infected subject by centrifugation at 50,000 xg for 80 minutes (m) at 4°. Viral RNA was extracted from the pellet using the PURE*script* Total RNA Isolation Kit (Gentra Systems). Briefly, 500 µl of Cell Lysis Solution containing 20 µg of glycogen as carrier (Roche Molecular Biochemicals) was added and the sample was heated to 65° for 5 minutes to ensure complete lysis. Protein and DNA was precipitated by adding 200 µl of Protein-DNA Precipitating Solution and incubating on ice for 5 minutes, and then removed from the solution by centrifugation at 20,000 ×g for 5 m. Viral RNA was precipitated from the supernatant by adding it to 600 µl of propan-2-ol and gently inverting 50 times. The RNA was pelleted at 20,000 xg for 15 minutes, washed with 600 µl 70% (v/v) ethanol, dried at room temperature, dissolved in 50 µl of nuclease-free water and stored at -80°.

Complementary DNA was synthesised with Sensiscript RT (Qiagen). Reaction mixtures consisted of (in a 50 µl volume): 1× Sensiscript reaction buffer, 500 µM each dNTP, 500 nM primer *INT-1,* 25 units Rnasin RNase inhibitor (Promega), 5% (v/v) DMSO, 2.5 µl Sensiscript RT and 25 µl RNA extract. The reaction was incubated at 37° for 1 hour to complete the cDNA synthesis. First round and nested PCR reaction were then carried out with *Platinum Pfx* DNA polymerase. First round PCR reactions consisted of (50µl volume): 4 µl 10x *Pfx* buffer, 1 µl 1 0 mM each dNTP, 0.8 µl 50 mM MgSO₄, 300 nM each of primers *R-1* and *INT-1, 1* µl *Platinum Pfx* polymerase and 30 µl cDNA. Thermal cycling was as follows: 94° for 2 minutes (m); 93° for 10 seconds (s), 65° for 30 s, 68° for 4 m 30 s (10 cycles); 93° for 10 s, 60° for 30 s, 68° for 4 m 30 s + 5 additional s for each consecutive cycle (25 cycles); 68° for 7 m. Nested reactions contained (in100 µl): 9 µl 10× *Platinum Pfx* buffer, 2.7µl 10 mM dNTP mix, 1:8 µl MgSO₄, 300 nM *INT-2* and *U5-1,* 2 µl *Platinum Pfx* polymerase and 10 µl of first round reaction. Thermal cycling was as follows: 94° for 2 minutes (m); 93° for 10 seconds (s), 60° for 30 s, 68° for 4 m 30 s (10 cycles); 93° for 10 s, 55° for 30 s, 68° for 4 m 30 s + 5 additional s for each consecutive cycle (25 cycles); 68° for 7 m.

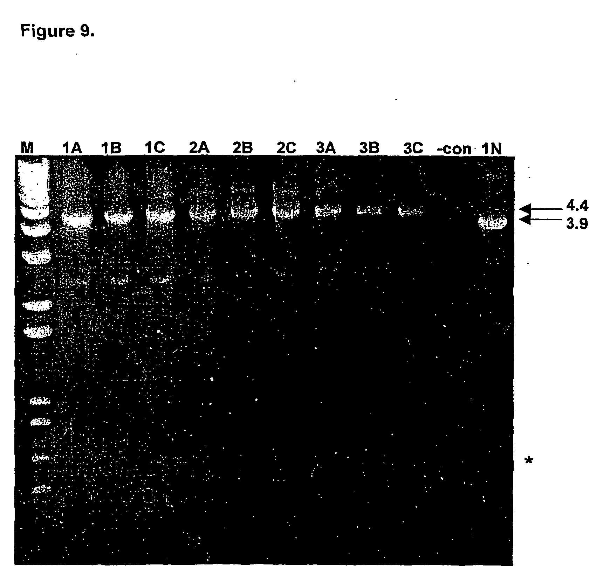

The LTR was added to the nested products by PMR prior to transfection. The PMR reactions successfully added the LTR sequences to the nested products (Fig. 9). The first round RT-PCR reactions were also used as templates to generate products for RVA by cotransfection with pHXBΔCSPRTC. Triplicate PCR products were cotransfected into MT4 cells with the linearised plasmid pHXBTOPO1, in the case of the PMR products, and with linearised pHXBΔCSPRTC in for the RVA products. Viral cytopathic effect was observed and titratable virus was harvested in the supernatants. No virus was isolated from virus 2 using FPH and only one of the triplicate cultures from virus 3 yielded infectious virus. All of the RVA cultures produced virus.

### Example 7: Recombinants generated by FPH can display lower drug susceptibility those generated by RVA

In order to determine whether the extra sequences in the FPH recombinants contain determinants of susceptibility, the recombinant viruses were tested against several drugs. Drug susceptibility was determined by the 3-(4,5-dimethylthiazol-2-yl)-2,5-diphenyltetrazolium bromide (MTT)-reduction colorimetric cell viability assay described previously (Pauwels *et al*. 1988; Robinson *et al.,* 2000). Changes in susceptibility were defined as fold increase in IC50 relative to WT (HXB2). Nucleotide sequences were determined using ABI 3700 capillary sequencers and Big Dye terminators. Susceptibility to all of the Pis tested and to the reverse transcriptase inhibitor zidovudine (ZDV) was lower in the FPH generated than the RVA generated viruses, as determined in two separate experiments (tables 3A and B, below). The largest difference observed was with ZDV, to which virus 1 displayed a 4.8-fold higher susceptibility when generated by RVA than when generated by FPH . Virus 1 also gave a 2.2-fold (Table 3A) or 4-fold (Table 3B) higher susceptibility to IDV and 2.9-fold (Table 3A) or 3.2-fold higher susceptibility to APV when generated by RVA. Virus 3 also displayed a difference in susceptibility between the two techniques, but less marked than with virus 1. Again, the largest difference was with ZDV (2.9 fold), the largest difference with the Pls was seen with APV (1.9 or 2.0-fold). Virus 2 obtained via the RVA showed high level resistance to both ZDV and APV (>84-fold and 16 or >21-fold higher than wild-type, respectively), consistent with the theory that it may have poor replicative capacity, and consistent with our inability to isolate it using FPH.

In this experiment we have demonstrated that recombinants generated by FPH can exhibit lower drug susceptibilities than those generated by a conventional method, due to the inclusion of additional sequence elements (in this case in gag) which are not represented in the standard RVA. This finding has important implications for the use of susceptibility phenotyping assays, since it implies that susceptibility in some isolates may previously have been underestimated. The application of the new assay should reveal new insights into the biology of drug susceptibility and enable more reliable estimations of drug efficacy.

**Table 2. Genotypes and therapy histories.**

| **Clinical Isolate** | **Protease genotype** | **RT genotype** | **Prior PI therapy** | **Weeks on PI therapy** | **Prior RTI therapy** |
|---|---|---|---|---|---|
| 1 | 154M | A62V, V75T, M184V | APV | 28 | 3TC, ZDV, d4T |
| 2 | L10I, M46I, I50V, L63R, A71T, | ND | APV | 79 | 3TC, ZDV, d4T |
| 3 | L10I, V321, M46L, L63P, A71V, V82A | M41 m/l, T69N, M184V | IDV, APV | 52 wks IDV, 8 wks APV | ddl, 3TC, ZDV, d4T |

| | | | | | |
|---|---|---|---|---|---|
| Note that virus from patient 3 was catalogued as having receiving only IDV, however, the genotyping and resistance profiling (data not shown) suggest that APV was the predominant therapy. | | | | | |

**Table 3A. Drug susceptibilities of recombinants generated from plasma by FPH or RVA**

| **Virus** | **Method** | **Drug EC50 in nM (fold resistance)** | | | | |
|---|---|---|---|---|---|---|
| | **RVA/FPH** | **APV** | **IDV** | **RTV** | **NFV** | **SQV** |
| 1 | FPH | 366.0(5.4) | 31.2(1.1) | 205.0(3.1) | 70.0 (2.3) | 13.1 (1.0) |
| | RVA | 128.0 (1.9) | 14.2 (0.48) | 90.4 (1.4) | 26.9 (0.90) | 6.56 (0.5) |
| 2 | FPH | ND | ND | ND | ND | ND |
| | RVA | 1070.0 (16) | 18.7(0.63) | 454.0 (7.0) | 70.8 (2:4) | 9.06 (0.69) |
| 3 | FPH | 718.0(11) | 1250(42) | 1790.0(27) | 591.0(20) | 16.4(1.3) |
| | RVA | 396.0 (5.9) | 689.0 (23) | 1400.0 (22) | 390.0 (13) | 13.7(1.0) |
| HXB2 | N/A | 67.5(1.0) | 29.7(1.0) | 65.1 (1.0) | 29.9(1.0) | 13.1(1.0) |

**Table 3B.**

| **Virus** | **Method** | **Drug EC50 in nM (fold resistance)** | | |
|---|---|---|---|---|
| | **RVA/ FPH** | **APV** | **IDV** | **ZDV** |
| 3.1 . | FPH | 865.0 (7.7) | 60.1 (2.0) | 76.1 (2.7) |
| | RVA | 211.0 (1.7) | 11.6 (-2.7) | 12.3 (-2.5) |
| 3.2 | FPH | 691.0 (6.1) | 57.1 (1.9) | 65.3 (2.3) |
| | RVA | 245.0 (2.0) | 16.5 (-1.9) | ND |
| 3.3 | FPH | 619.0 (5.5) | 53.2 (1.7) | 35.1 (1.2) |
| 4.1 | RVA | >2500 (>21) | 65.0 (2.1) | >5000 (>163) |
| 4.2 | RVA | 2010.0 (17) | 23.8 (-1.3) | >5000 (>163) |
| 4.3 | RVA | >2500 (>21) | 39.3(1.3) | >5000 (>163) |
| 5.1 | FPH | 1570.0 (14) | 2230.0 (72) | 48.2 (1.7) |
| 5.2 | RVA | 802.0 (6.6) | 1520.0 (49) | 16.5 (-1.9) |
| WT | FPH | 113.0 (1,0) | 30.8 (1.0) | 28.3 (1.0) |
| | RVA | 121.0 (1.0) | 31.2 (1.0) | 30.7 (1.0) |

### References

Alford, J.L. et al. (1999). Antiviral Therapy 4 (Suppl 1):30.
Back, N.K.T. et al. (1996). EMBO J 15:4040-4049.
Baffour Jr, H.H. (1999). Drug Therapy 340 (16):1255-1268.
Bames, W.M. (1992). Gene 112: 29-35.
Barnes, W.M. (1994). Proc Natl Acad Sci USA 91: 2216-2220.
Carriilo, A. et al. (1998). J. Virol. 72:7532-7541.
Coffin, J.M. (1995). Science 267:483-489.
Doyon, L., et al. (1996). J. Virol. 70:3763-3769.
Fang, G, et al. (1999). Nature Medicine 5(2):239-242.
Hertogs, K. et al. (1998). Antimicrob. Agents Chemother. 42:269-276.
Ho, D.D. et al. (1995). Nature 373:123-126.
Japour, A.J. et al. (1993). Antimicrob. Agents Chemother. 37:1095-1101.
Kellam, P. and B.A. Larder. (1994). J. Virol. 38:23-30.
Kimberlin, D.W. and R.J. Whitley. (1996). J. Antimicrobial Chemotherapy 37:403-421.
Kusumi, K. et al. (1992). J. Virol. 66:875-885.
Larder, B.A. et al. (1989). Proc. Natl. Acad. Sci. USA 86:4803-4807.
Lundberg, K.S. et al. (1991). Gene 108: 1-6.
Mammano, F. et al. (1998). J. Virol. 72:7632-7637.
Mansky, L.M. and H.M. Temin. (1995). J. Virol. 69:5087-5094.
Maschera, B. et al. (1995). J. Virol. 69:5431-5436.
Maschera, B. et al. (1996a). J. Biol. Chem. 271:33231-33235.
Maschera, B., et al. (1996b). abstr., p. 85. in Programme and Abstracts of the
5^{th} International Workshop on HIV Drug Resistance 1996.
Mayers, D. L., et al. 1998. abstr. No. 62, p. 42. in Programme and Abstracts of the 2^{nd} International Workshop on HIV. Drug Resistance and Treatment Strategies 1998.
Pauwels, R. et al. (1988) J. Virol. Methods 20: 309-321.
Pazhanismay, S. et.al. (1998) in James (Ed.), Aspartic Proteinases - Plenum Press, New York.
Robinson, L.H. et al. (1999) British Patent Application No. 9909793.3
Robinson, L.H. et al. (2000) AIDS Res. Hum. Retr. 16: 1149-1156.
Vandamme, A.-M., et al. (1999). Drugs 57(3)337-361.
Wei, X., et al. (1995). Nature 373:117-122.
Zennou, V., et al. (1998). J. Virol. 72:3300-3306.
Zhang, Y.-M., et al. (1997). J. Virol. 71:6662-6670.
Martinez-Picado et al. (1999). J. Clim. Microbiol. 37:2943-2951.

### Figure Legends:

### FIGURE 3. Plasmid clone of 5' half of the HXB2 provirus.

Plasmid p5'HXB2 was constructed by cloning an LTR-1/INT-1 PCR product from MT4 cell DNA infected with wild type HXB2 into the pCR2.1 TOPO vector (Invitrogen). The proofreading DNA polymerase *Pfx* was used for the amplification (Gibco BRL). Plasmid can be linearised with *Xba*l prior to cotransfection with DNA to produce virus.

### FIGURE 4. Plasmid clone of 3' half of the HXB2 provirus.

Plasmid pHXBTOPO1 was constructed by cloning an INT-F / PIB13'CS PCR product of the RVA vector pHXBΔPR (Maschera *et al*., 1995), into the pCR2.1TOPO vector (Invitrogen). The proofreading DNA polymerase *Pfu* was used for the amplification (Stratagene). Plasmid was linearised with *Spe*l prior to cotransfection with RT-PCR products.

### FIGURE 6. Plasmid clone of U3-R megaprimer.

Plasmid pFPHmegaprim1 was constructed by cloning an LTR-1 /BsaHl(L TR-R) PCR product of the RVA vector pHXBΔPR (Maschera, *et al.,* 1995), into the pCR2.1TOPO vector (Invitrogen). The proofreading DNA polymerase *Pfu* was used for the amplification (Stratagene). The megaprimer can be generated by PCR amplification of digestion with *Bam*HI and *Bsa*HI.

### FIGURE 9. FPH PCR-mediated recombination (PMR) products (Example 6).

- M =: 1 kB Plus DNA size marker (Gibco BRL)
- 1 N =: nested product of isolate 1
- * =: 634-bp megaprimer
- -con =: negative control

Sizes are indicated in kilobase pairs.

## Claims

1. A method for producing recombinant virus comprising recombination of a nudeotide sequence derived from a virus sample with a backbone sequence from a standard virus strain to produce recombinant virus, wherein the backbone sequence comprises a portion of the infectious nucleic acid sequence of a laboratory virus strain extending from a first site within or adjacent to the 3' or the 5' terminus of the infectious nucleic acid sequence, and the nucleotide sequence derived from the virus sample comprises the remainder of the infectious nucleic acid sequence.

2. An assay for the detection and characterisation in a virus sample of virus resistant to an anti-viral drug, the assay comprising the steps of;
(i) producing recombinant virus having the resistance profile of the virus sample by recombination of a nucleotide sequence derived from the virus sample with a backbone sequence from a standard virus strain, wherein the backbone sequence comprises a portion of a laboratory virus strain infectious nucleic acid sequence extending from a first site within or adjacent to the 3' or the 5' terminus of the infectious nucleic acid sequence, and the nucleotide sequence derived from the virus sample comprises the remainder of the infectious nucleic acid sequence;
(ii) incubating cells infected with the recombinant virus with an antiviral drug; and
(iii) detecting the viability of virus or of infected cells after incubation, to determine the sensitivity of the recombinant virus to the drug.

3. An assay according to claim 2 wherein the backbone sequence comprises 20-90% of the laboratory virus strain infectious nucleic acid sequence.

4. An assay according to claim 3 wherein the backbone sequence comprises 25-75% of the laboratory virus strain infectious nucleic acid sequence.

5. An assay according to claim 4 wherein the backbone sequence comprises at least one third of the laboratory virus strain infectious nucleic acid sequence.

6. An assay according to claim 5 wherein the backbone sequence comprises approximately half of the laboratory virus strain infectious nucleic acid sequence.

7. An assay according to claim 6 wherein the backbone sequence comprises the 3' half of the laboratory virus strain infectious nucleic acid sequence.

8. An assay according to any one of claims 2 to 7 wherein the virus is HIV-1 and the backbone sequence is derived from a wild type HIV-1 DNA provirus.

9. An assay according to claim 8 wherein the DNA backbone sequence includes a sequence from within the integrase gene and extending to the 3' end of the 3' LTR.

10. A method according to claim 1 wherein the backbone sequence comprises 20-90% of the laboratory virus strain infectious nucleic acid sequence.

11. A method according to claim 10 wherein the backbone sequence comprises 25-75% of the laboratory virus strain infectious nucleic acid sequence.

12. A method according to claim 11 wherein the backbone sequence comprises at least one third of the laboratory virus strain infectious nucleic acid sequence.

13. A method according to claim 12 wherein the backbone sequence comprises approximately half of the laboratory virus strain infectious nucleic acid sequence.

14. A method according to claim 13 wherein the backbone sequence comprises the 3' half of the laboratory virus strain infectious nucleic acid sequence.

15. A method according to claim 1 or any one of claims 10 to 14 wherein the virus is HIV-1 and the backbone sequence is derived from a wild type HIV-1 DNA provirus.

16. A method according to claim 15 wherein the DNA backbone sequence includes a sequence from within the integrase gene and extending to the 3' end of the 3' LTR.

17. A method according to claim 15 or 16 wherein the DNA backbone sequence includes the *env* gene.

18. A method according to claim 1 wherein the sequence derived from the virus sample includes the sequence encoding the protease drug target.

19. A method according to claim 18 wherein the sequence derived from the virus sample includes the *gag* sequence.

20. A method according to claim 18 or claim 19 wherein the sequence derived from the virus sample includes the sequences encoding the protease and reverse transcriptase drug targets and all of the protease cleavage sites in Gag and Pol.

21. Use of a backbone sequence comprising sequences extending from a first site at or adjacent the 3' or the 5' terminus of a laboratory virus strain infectious nucleic acid sequence to a second site within the laboratory virus strain infectious nucleic acid sequence in a method according to any one of claims 1 and 10-20 for producing recombinant virus.

22. Use of a backbone sequence comprising sequences extending from a first site at or adjacent the 3' or the 5' terminus of a laboratory virus strain infectious nucleic acid sequence to a second site within the laboratory virus strain infectious nucleic acid sequence in an assay according to any one of claims 2-9 for the detection of virus resistant to an anti-viral drug.

23. Use according to claim 21 or claim 22 wherein the backbone sequence comprises the 3' end of the laboratory virus strain infectious nucleic acid sequence.

24. Use according to claim 23 wherein the backbone sequence comprises at least 25% of the infectious nucleic acid sequence.

25. Use according to claim 23 or claim 24 wherein the backbone sequence comprises at least one third of the infectious nucleic acid sequence.

26. Use according to any one of claims 23 to 25 wherein the backbone sequence comprises approximately half of the laboratory virus strain infectious nucleic acid sequence.

27. Use according to any one of claims 23 to 26 wherein the laboratory virus strain is wild-type HIV-1 and the sequence comprises the 3' half of the HIV provirus.

28. Use according to any one of claims 23 to 27 wherein the DNA backbone sequence includes a sequence extending from within the integrase gene to the 3' end of the 3' LTR.

## Patentansprüche

1. Verfahren zur Herstellung von rekombinantem Virus, umfassend lie Rekombination einer Nucheo2idsequenz abgeleitet von einer Virusprobe mit einer auptsequenz eines Standardvirusstammes, um ein rekombinantes Virus herzustellen, wobei die Hauptsequenz einen Teil der infektiösen Nucleinsäuresequenz eines Laborvirusstammes umfasst, der sich von einer ersten Stelle innerhalb oder angrenzend n den 3'- oder 5'-Terminus der infektiösen Nucleinsäuresequenz erstreckt, und die Nucleotidsequenz abgeleitet von der Virusprobe den Rest der infektiösen Nucleinsäuresequenz umfasst.

2. Test zum Nachweisen und Charakterisieren von Viren in einer Virusprobe, die resistent gegen einen antiviralen Arzneistoff sind, wobei der Test die Schritte umfasst:
(i) Herstellung von rekombinantem Virus, der das Resistenzprofil der Virusprobe hat, durdh Rekombination einer Nucleotidsequenz abgeleitet on der Virusprobe mit einer Hauptsequenz eines Standardvirusstammes, wobei die Hauptsequenz einen Teil der infektiösen Nucleinsäuresequenz eines aborvirusstammes umfasst, der sich von einer ersten Stelle innerhalb oder ang enzend an den 3'-oder den 5'-Terminus der infektiösen Nucleinsäuresequenz erstreckt, und die Nucleotidsequenz abgeleitet von der Virusprobe den Rest der infektiösen Nucleinsäuresequenz umfasst;
(ii) Inkubation der Zellen, die mit dem rekombinanten Virus infiziert sind, mit einem antiviralen Arzneistoff; und
(iii) Nachweis der Lebensfähigkeit des Virus oder der infizierten Zellen nach Inkubation, um die Sensitivität des rekombinanten Virus auf den Arzneistoff zu bestimmen.

3. Test nach Anspruch 2, wobei die Hauptsequenz 20 bis 90% der infektiösen Nucleinsäuresequenz des Laborvirusstammes umfasst.

4. Test nach Anspruch 3, wobei die Hauptsequenz 25 bis 75% der infektiösen Nucleinsäuresequenz des Laborvirusstammes umfasst.

5. Test nach Anspruch 4, wobei die Hauptsequenz mindestens ein Drittel der infektiösen Nucleinsäuresequenz des Laborvirusstammes umfasst.

6. Test nach Anspruch 5, wobei die Hauptsequenz ungefähr die Hälfte der infektiösen Nucleinsäuresequenz des Laborvirusstammes umfasst.

7. Test nach Anspruch 6, wobei die Hauptsequenz die 3'-Hälfte der infektiösen Nucleinsäuresequenz des Laborvirusstammes umfasst.

8. Test nach einem der Ansprüche 2 bis 7, wobei das Virus HIV-1 ist und die Hauptsequenz von einem Wildtyp HIV-1-DNA-Provirus abgeleitet ist

9. Test nach Anspruch 8, wobei die DNA-Hauptsequenz eine Sequenz von innerhalb des Integrasegens einschließt und sich bis zum 3'-Ende der 3'-LTR erstre kt.

10. Verfahren nach Anspruch 1, wobei die Hauptsequenz 20 bis 90% der infektiösen Nucleinsäuresequenz des Laborvirusstammes umfasst.

11. Verfahren nach Anspruch 10, wobei die Hauptsequenz 25 bis 75% der infektiösen Nucleinsäuresequenz des Laborvirusstammes umfasst.

12. Verfahren nach Anspruch 11, wobei die Hauptsequenz mindestens ein Drittel der infektiösen Nucleinsäuresequenz des Laborvirusstammes umfasst.

13. Verfahren nach Anspruch 12, wobei die Hauptsequenz ungefaür die Hälfte der infektiösen Nucleinsäuresequenz des Laborvirusstammes umfasst.

14. Verfahren nach Anspruch 13, wobei die Hauptsequenz die 3'-Hälfte der infektiösen Nucleinsäuresequenz des Laborvirusstammes umfasst.

15. Verfahren nach Anspruch 1 oder einem der Ansprüche 10 bis 14, wobei das Virus HIV-1. ist und die Hauptsequenz von einem Wildtyp HIV-1-DNA-Provirus abgeleitet ist.

16. Verfahren nach Anspruch 15, wobei die DNA-Hauptsequenz eine Sequenz von innerhalb des Integrasegens einschließt und sich bis zum 3'-Ende der 3'-LTR erstreckt.

17. Verfahren nach Anspruch 15 oder 16, wobei die DNA-Hauptsequenz das *env*-Gen einschließt.

18. Verfahren nach Anspruch 1, wobei die Sequenz, die von der Virusprobe abgeleitet ist, die Sequenz einschließt, die den Arzneistoff-Angriffspunkt der Protease codiert.

19. Verfahren nach Anspruch 18, wobei die Sequenz, die von der Virusprobe abgeleitet ist, die *gag*-Sequenz einschließt.

20. Verfahren nach Anspruch 18 oder 19, wobei die Sequenz, die von der Virusprobe abgeleitet ist, die Sequenzen einschließt die die Protease- und Reverse-Transkriptase-Arzneistoff-Angriffspunkte und alle Protease-Schnittstellen in Gag und Pol codieren.

21. Verwendung einer Hauptsequenz, umfassend Sequenzen, die sich von einer ersten Stelle auf oder angrenzend an den 3'- oder den 5'-Terminus der infektiösen Nucleinsäuresequenz eines Laborvirusstammes bis zu einer zweiten Stelle innerhalb der infektiösen Nucleinsäuresequenz des Laborvirusstammes erstrecken, an einem Verfahren nach einem der Ansprüche 1 und 10 bis 20 zur Herstellung von rekorbinantem Virus.

22. Verwendung einer Hauptsequenz, umfassend die Sequenzen, die sich von einer ersten Stelle auf oder angrenzend an den 3'- oder den 5'-Terminus der infektiösen Nucleinsäuresequenz eines Laborvirusstammes bis zu einer zweiten Stelle innerhalb der infektiösen Nucleinsäuresequenz des Laborvirusstammes erstrecken an einem Test nach einem der Ansprüche 2 bis 9 zum Nachweis eines Virus, der gegenüber einem antiviralen Arzneistoff resistent ist.

23. Verwendung nach Anspruch 21 oder Anspruch 22, wobei die Hauptsequenz das 3'-Ende der infektiösen Nucleinsäuresequenz des Laborvirusstammes umfasst.

24. Verwendung nach Anspruch 23, wobei die Hauptsequenz mindestens 25% der infektiösen Nucleinsäuresequenz umfasst.

25. Verwendung nach Anspruch 23 oder Anspruch 24, wobei die Hauptsequenz mindestens ein Drittel der infektiösen Nucleinsäuresequenz umfasst.

26. Verwendung nach einem der Ansprüche 23 bis 25, wobei die Hauptsequenz ungefähr die Hälfte der infektiösen Nucleinsäuresequenz des Laborvirusstammes umfasst.

27. Verwendung nach einem der Ansprüche 23 bis 26, wobei der Laborvirusstamm der HIV-1-Wildtyp ist und die Sequenz die 3'-Hälfte des HTV-Provirus umfasst

28. Verwendung nach einem der Ansprüche 23 bis 27, wobei die DNA Hauptsequenz eine Sequenz einschließt, die sich von innerhalb des Integrasegens bis zum 3'-Ende der 3'-LTR erstreckt.

## Revendications

1. Procédé pour la production d'un virus recombinant, comprenant la recombinaison d'une séquence de nucléotides dérivée d'un échantillon de virus avec une séquence de squelette provenant d'une souche de virus classique pour produire un virus recombinant, dans lequel la séquence de squelette comprend une partie de la séquence d'acide nucléique infectieuse d'une souche de virus de laboratoire s'étendant d'un premier site dans ou en position adjacente à l'extrémité 3' ou 5' de la séquence d'acide nucléique infectieuse, et la séquence de nucléotides dérivée de l'échantillon de virus comprend le reste de la séquence d'acide nucléique infectieuse.

2. Analyse pour la détection et la caractérisation dans un échantillon de virus d'un virus résistant à un médicament antiviral, l'analyse comprenant les étapes consistant :
(i) à produire un virus recombinant présentant le profil de résistance de l'échantillon de virus par recombinaison d'une séquence de nucléotides dérivée de l'échantillon de virus avec une séquence de squelette provenant d'une souche de virus classique, où la séquence de squelette comprend une partie d'une souche d'une séquence d'acide nucléique infectieuse de souche de virus de laboratoire s'étendant d'un premier site dans ou en position adjacente à l'extrémité 3' ou 5' de la séquence d'acide nucléique infectieuse, et la séquence de nucléotides dérivée de l'échantillon de virus comprend le reste de la séquence d'acide nucléique infectieuse ;
(ii) à mettre en incubation des cellules infectées avec le virus recombinant avec un médicament antiviral ; et
(iii) à détecter la viabilité du virus ou des cellules infectées après incubation, pour déterminer la sensibilité du virus recombinant au médicament.

3. Analyse suivant la revendication 2, dans laquelle la séquence de squelette comprend 20 à 90 % de la séquence d'acide nucléique infectieuse de la souche de virus de laboratoire.

4. Analyse suivant la revendication 3, dans laquelle la séquence de squelette comprend 25 à 75 % de la séquence d'acide nucléique infectieuse de la souche de virus de laboratoire.

5. Analyse suivant la revendication 4, dans laquelle la séquence de squelette comprend au moins un tiers de la séquence d'acide nucléique infectieuse de la souche de virus de laboratoire.

6. Analyse suivant la revendication 5, dans laquelle la séquence de squelette comprend approximativement la moitié de la séquence d'acide nucléique infectieuse de la souche de virus de laboratoire.

7. Analyse suivant la revendication 6, dans laquelle la séquence de squelette comprend la moitié 3' de la séquence d'acide nucléique infectieuse de la souche de virus de laboratoire.

8. Analyse suivant l'une quelconque des revendications 2 à 7, dans laquelle le virus est le HIV-1 et la séquence de squelette est dérivée d'un provirus à ADN de HIV-1 de type sauvage.

9. Analyse suivant la revendication 8, dans laquelle la séquence de squelette d'ADN comprend une séquence provenant de l'intérieur du gène d'intégrase et s'étendant à l'extrémité 3' de la 3' LTR.

10. Procédé suivant la revendication 1, dans lequel la séquence de squelette comprend 20 à 90 % de la séquence d'acide nucléique infectieuse de la souche de virus de laboratoire.

11. Procédé suivant la revendication 10, dans lequel la séquence de squelette comprend 25 à 75 % de la séquence d'acide nucléique infectieuse de la souche de virus de laboratoire.

12. Procédé suivant la revendication 11, dans lequel la séquence de squelette comprend au moins un tiers de la séquence d'acide nucléique infectieuse de la souche de virus de laboratoire.

13. Procédé suivant la revendication 12, dans lequel la séquence de squelette comprend approximativement la moitié de la séquence d'acide nucléique infectieuse de la souche de virus de laboratoire.

14. Procédé suivant la revendication 13, dans lequel la séquence de squelette comprend la moitié 3' de la séquence d'acide nucléique infectieuse de la souche de virus de laboratoire.

15. Procédé suivant la revendication 1 ou suivant l'une quelconque des revendications 10 à 14, dans lequel le virus est le HIV-1 et la séquence de squelette est dérivée d'un provirus à ADN de HIV-1 de type sauvage.

16. Procédé suivant la revendication 15, dans lequel la séquence de squelette d'ADN comprend une séquence provenant de l'intérieur du gène d'intégrase et s'étendant à l'extrémité 3' de la LTR 3'.

17. Procédé suivant la revendication 15 ou 16, dans lequel la séquence de squelette d'ADN comprend le gène env.

18. Procédé suivant la revendication 1, dans lequel la séquence dérivée de l'échantillon de virus comprend la séquence codant pour la cible de médicament protéase.

19. Procédé suivant la revendication 18, dans lequel la séquence dérivée de l'échantillon de virus comprend la séquence *gag.*

20. Procédé suivant la revendication 18 ou la revendication 19, dans lequel la séquence dérivée de l'échantillon de virus comprend la séquence codant pour les cibles de médicament protéase et transcriptase inverse et la totalité des sites de clivage de protéases dans Gag et Pol.

21. Utilisation d'une séquence de squelette comprenant des séquences s'étendant d'un premier site à la position adjacente de l'extrémité 3' ou 5' d'une séquence d'acide nucléique infectieuse d'une souche de virus de laboratoire à un second site dans la séquence d'acide nucléique infectieuse de la souche de virus de laboratoire dans un procédé suivant l'une quelconque des revendications 1 et 10 à 20 pour la production d'un virus recombinant.

22. Utilisation d'une séquence de squelette comprenant des séquences s'étendant d'un premier site à ou en position adjacente à l'extrémité 3' ou 5' d'une séquence d'acide nucléique infectieuse d'une souche de virus de laboratoire à un second site dans la séquence d'acide nucléique infectieuse de la souche de virus de laboratoire dans une analyse suivant l'une quelconque des revendications 2 à 9 pour la détection d'un virus résistant à un médicament antiviral.

23. Utilisation suivant la revendication 21 ou la revendication 22, dans laquelle la séquence de squelette comprend l'extrémité 3' de la séquence d'acide nucléique infectieuse de la souche de virus de laboratoire.

24. Utilisation suivant la revendication 23, dans laquelle la séquence de squelette comprend au moins 25 % de la séquence d'acide nucléique infectieuse.

25. Utilisation suivant la revendication 23 ou la revendication 24, dans laquelle la séquence de squelette comprend au moins un tiers de la séquence d'acide nucléique infectieuse.

26. Utilisation suivant l'une quelconque des revendications 23 à 25, dans laquelle la séquence de squelette comprend approximativement la moitié de la séquence d'acide nucléique infectieuse de la souche de virus de laboratoire.

27. Utilisation suivant l'une quelconque des revendications 23 à 26, dans laquelle la souche de virus de laboratoire est le HIV-1 de type sauvage, et la séquence comprend la moitié 3' du provirus de HIV.

28. Utilisation suivant l'une quelconque des revendications 23 à 27, dans laquelle la séquence de squelette d'ADN comprend une séquence s'étendant de l'intérieur du gène d'intégrase à l'extrémité 3' de la 3' LTR.
